(19)

Europäisches
Patentamt
European
Patent Office
Office européen
des brevets

(11) **EP 4 520 267 A1**

(12) **EUROPEAN PATENT APPLICATION**

(43) Date of publication:
**12.03.2025 Bulletin 2025/11**

(21) Application number: **23196082.4**

(22) Date of filing: **07.09.2023**

(51) International Patent Classification (IPC):
*A61B 5/18* (2006.01)          *A61B 5/372* (2021.01)

(52) Cooperative Patent Classification (CPC):
**A61B 5/18; A61B 5/372**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicants:
• **TOYOTA JIDOSHA KABUSHIKI KAISHA**
**Toyota-shi, Aichi-ken, 471 8571 (JP)**
• **UCL Business Ltd**
**London WC1E 6BT (GB)**

(72) Inventors:
• **ABDELKAWY, Hazem**
**1140 BRUSSELS (BE)**
• **AMBECK-MADSEN, Jonas**
**1140 BRUSSELS (BE)**
• **LAVIE, Nilli**
**London, N8 9HU (GB)**
• **BARNE, Louise Catheryne**
**London, NW5 4AY (GB)**

(74) Representative: **Cabinet Beau de Loménie**
**158, rue de l'Université**
**75340 Paris Cedex 07 (FR)**

Remarks:
Amended claims in accordance with Rule 137(2)
EPC.

(54)     **METHOD AND DEVICE FOR DETERMINING AN ATTENTION LEVEL OF A PERSON**

(57)     The present invention relates to a method and device for determining an attention level of a person, and also relates to a driving assistance method and system, a vehicle, a computer program, and a computer-readable medium.

The proposed method for determining the attention level (LVL) is based on signals (EEG) representative of a neural activity of the person, and comprises:
- a step of estimating (S220) parameters ($\theta_N$) of power spectra ($S_N$) of the neural activity signals (EEG) over one time interval ($I_N$), the estimated parameters ($\theta_N$) including power-law exponents ($\chi_N$) and/or offsets ($b_N$) of the power spectra ($S_N$); and
- a step of determining (S230) the attention level (LVL) based on a variation between: the parameters ($\theta_N$) estimated for said time interval ($I_N$); and parameters ($\theta_1$) estimated for a preceding time interval ($I_1$).

EEG $I_1$-$I_N$

**S200** DET LVL

**S210** OBT $S_1$, $S_N$

**S220** EST $\theta_1$ [$\chi_1$, $b_1$, $P_1$] , $\theta_N$ [$\chi_N$, $b_N$, $P_N$]

**S230** DET LVL ($\theta_1$ , $\theta_N$)

LVL

**FIG. 9**

EP 4 520 267 A1

## Description

BACKGROUND OF THE INVENTION

1. Field of the invention

The present invention relates to a method and device for determining an attention level of a person, and also relates to a driving assistance method and system, a vehicle, a computer program, and a computer-readable medium.

2. Description of Related Art

The present invention lies, in particular, within the context of driving assistance systems. These systems are designed to assist drivers (*i.e.*, humans) in operating a vehicle and, among other things, to improve safety and prevent accidents.

Since drivers' fatigue and distraction are major contributors to accidents, some driver assistance systems monitor the driver's attention level (*i.e.,* his/her vigilance level, alertness). Such systems use various types of information, such as images captured by cameras, physiological signals from the driver, or the vehicle behavior, to determine whether the driver is alert and capable of driving safely. If not, the driver assistance system can alert the driver, or even perform a safety maneuver if necessary.

To monitor the driver's attention level, some driving assistance systems use cameras and infrared sensors to track the driver's eye movements. For example, a high blink frequency or extended periods of eye closure are signs of the driver's fatigue. However, these systems require a significant period of time to detect the driver's fatigue, and could be too late in warning the driver to prevent an accident.

It has also been proposed to use machine learning algorithms in driving assistance systems to monitor a driver's attention level. These algorithms are typically trained to evaluate a driver's attention level using images captured by on-board cameras, or physiological signals from the driver, or even the vehicle behavior as input data. However, these systems require input data measured over a significant period of time to determine the driver's attention level.

Existing driving assistance systems are not fully satisfactory. They require a significant period of time to evaluate a driver's attention level. Therefore, there exists a need for a solution that allows determining a driver's attention level in real-time.

SUMMARY

The present invention has been made in light of the above problems.

According to an aspect, the present invention provides a (computer-implemented) method for determining at least one attention level of a person based on time signals (referred to as the *neural activity signals*) representative of a neural activity of the person over a plurality of time intervals, the method comprising for at least one said time interval:

- a step of estimating parameters of power spectra of the neural activity signals over said time interval, the estimated parameters including power-law exponents and/or offsets of the power spectra; and
- a step of determining a said attention level of the person based on a variation between: the parameters estimated for said time interval (including said power-law exponents and/or offsets); and parameters estimated for a preceding time interval (including power-law exponents and/or offsets).

The method proposed here uses the variation of the power-law exponents (*i.e.*, the slope of the 1/f characteristic) of the power spectra of the neural activity signals as a marker of a person's attention. A fluctuation in a person's attention with the time spent on a task is associated with a variation of the slope of the 1/f characteristic. As such, a variation of the slope of the 1/f characteristic over time is a relevant marker of a fluctuation of a person's attention. This marker allows detecting rapidly (within 800 milliseconds) the fluctuations of a person's attention. In addition, the slope of the 1/f aperiodic component is a reliable marker of attention, since it is unaffected by inter-individual differences related to alpha peak frequency.

Alternatively to, or in combination with, the power law exponents, the proposed method uses the variation of the offsets (*i.e.*, the offset of the 1/f characteristic) of the power spectra of neural activity signals as a marker of a person's attention. A fluctuation in a person's attention with time-on-task is associated with a variation of the offset of the 1/f characteristic. In other words, a variation of the offset of the 1/f characteristic over time is a relevant marker of a fluctuation of a person's attention, and allows detecting rapidly such fluctuation (within 800 milliseconds).

Thereby, the present invention allows advantageously determining a person's attention level in real-time and reliably.

According to an embodiment, said attention level of the person is determined to be a reduced attention level if the following condition is satisfied:

- all or part of the power-law exponents estimated for said time interval are larger than all or part of power-law exponents estimated for said preceding time interval, and in particular a visual scene perceived by the person is of high perceptual load (superior to a threshold).

This embodiment proposes to detect a decrement in the person's attention level if the power-law exponents (*i.e.*, the slope of the 1/f characteristic) of the power spectra of the neural activity signals increase over time and the visual scene perceived by the person (*i.e., the driving scene*) is of high perceptual load. The inventors' experimental results have shown that: in conditions of high perceptual load, a person's attention level decreases with time on task, and this decrement in attention is associated with a steeper slope of the 1/f characteristic. This embodiment hence allows detecting in real-time a reduction in a person's attention level.

Examples of high perceptual load scenes include urban driving scenes, and driving scenes involving vehicles and pedestrians in motion.

According to an embodiment, said attention level of the person is determined to be a reduced attention level if the following condition is satisfied:

- all or part of the offsets estimated for said time interval are larger than all or part of offsets estimated for said preceding time interval.

In this embodiment, it is proposed to detect a decrement in the person's attention if the offsets (*i.e.*, the offset of the 1/f characteristic) of the power spectra of the neural activity signals increase over time. The inventors have found that: in conditions of high and low perceptual load, a person's attention level decreases with time on task, and this decrement in attention is associated with a larger offset of the 1/f characteristic. This embodiment allows detecting in real-time a reduction in a person's attention level.

According to an embodiment, the estimated power-law exponents and/or offsets used to determine said attention level correspond to power-law exponents and/or offsets of power spectra of the neural activity signals in the right central-parietal region.

As previously mentioned, the inventors have found that: in conditions of high perceptual load, a variation of the power-law exponents (*i.e.*, the slope of the 1/f characteristic) of the power spectra of the neural activity signals over time is a particularly relevant marker of a fluctuation of a person's attention. The inventors have also found that: a variation of the offsets (*i.e.*, the offset of the 1/f characteristic) of the power spectra of the neural activity signals over time is a relevant marker of a fluctuation of a person's attention (regardless of the perceptual load). They have shown in particular that the variation of the slope and the offset of the 1/f characteristic is most pronounced for the neural activity signals measured in the right central-parietal region. This embodiment is particularly advantageous in that it limits the number of sensors and processing operations required to determine a person's attention level in real-time and reliably.

According to an embodiment, the estimated parameters are parameters of models of the power spectra such that each power spectrum is modelled as a combination of an aperiodic component and zero or more periodic components, and that the aperiodic component is defined by one of the estimated power-law exponents and one of the estimated offsets.

Neural activity signals are representative not only of periodic activity, but also of aperiodic activity. These periodic and aperiodic components overlap and vary over time, such that a variation in the neural activity signals can be related to a variation in either or both of these components. By modeling power spectra into aperiodic and periodic components - which is detailed hereinafter, this embodiment allows to separate these components and to measure their variations independently. It hence allows estimating accurately the power-law exponent (*i.e.*, the slope of the 1/f aperiodic component) and the offsets (*i.e.,* the offset of the 1/f aperiodic component), and measuring the variation of these parameters over time. This embodiment contributes to determining reliably a person's attention level based on a variation of the slope and/or the offset of the 1/f characteristic.

According to an embodiment, the estimated parameters further include powers of peaks in the alpha band (*i.e.*, 8-12 Hz) of the power spectra. More specifically, in this embodiment, the attention level of the person is determined using jointly:

- a variation between: i) the power-law exponents and/or the offsets estimated for said time interval, and ii) power-law exponents and/or offsets estimated for said preceding time interval; and

- a variation between the powers of the peaks in the alpha-band estimated for said time interval and powers of peaks in the alpha-band estimated for said preceding time interval.

In addition to the above-mentioned experimental results, the inventors have shown that a fluctuation in a person's attention with time on task is also associated with variation in the power of the peak in the alpha-band. The inventors have shown, by parameterizing the power spectrum into aperiodic and periodic components, that time-on-task had an effect on alpha oscillations, and that this effect was not due to the underlying aperiodic activity. Hence, using the power of the peak in the alpha band after the aperiodic component is modelled allows improving the reliability of the proposed solution for determining a person's attention level.

According to an embodiment, said attention level of the person is determined to be a reduced attention level if all of the following conditions are jointly satisfied:

- all or part of the power-law exponents estimated for said time interval are larger than all or part of power-law exponents estimated for said preceding time interval, and in particular a visual scene perceived by the person is of high perceptual load;
- all or part of the offsets estimated for said time interval are larger than all or part of offsets estimated for said preceding time interval; and

- all or part of the powers of the peaks in the alpha-band estimated for said time interval are larger than all or part of powers of peaks in the alpha-band estimated for said preceding time interval.

As previously mentioned, a decrement in a person's attention level with time on task is associated with: a steeper slope of the 1/f characteristic in conditions of high perceptual load, and a larger offset of the 1/f characteristic regardless of the perceptual load. In addition, the inventors' experimental results show that the decrement in attention is further associated with a larger power of the peak in the alpha-band, regardless of the perceptual load. In this embodiment, a steeper slope of the 1/f characteristic (in conditions of high perceptual load), a larger offset of the 1/f characteristic, and a larger power of the peak of the alpha-band are jointly used as markers of an attention decline. This embodiment thereby allows to detect a reduction in a person's attention level in real-time and reliably.

According to an embodiment, the estimated powers of peaks in the alpha band used to determine said attention level correspond to powers of peaks in the alpha band of power spectra of the neural activity signals in the parieto-occipital region.

In particular, the inventors have found that a fluctuation of a person's attention is associated with a variation in the power of the alpha oscillations across all cortical regions. Nevertheless, the experimental results have shown that the power of alpha oscillations over parieto-occipital electrodes significantly predicted a person's accuracy in performing a task - which is detailed hereinafter. Accordingly, this embodiment proposes to determine the person's attention level using in particular the power of alpha oscillations measured in the parieto-occipital region (in addition to the slope and/or the offset of 1/f aperiodic component over all or part of the cortical regions). This embodiment is advantageous in that it limits the number of sensors and processing operations required to determine in real-time and reliably a person's attention level.

According to an embodiment, said step of estimating parameters of power spectra and said step of determining a said attention level are iterated at each of a plurality of time steps, and wherein each time step is at least 800 milliseconds long.

This embodiment provides a real-time implementation of the proposed method. Specifically, the person's attention level is determined every 800 milliseconds (or more). This allows monitoring the person's attention level in real-time. And, since the proposed method is tracking the proper markers of attention, it can rapidly detect (within 800 milliseconds) fluctuations in the person's attention level.

According to an embodiment, each said time interval is at least 2 minutes long and comprises multiple time slots and wherein each said time slot is at least 800 milliseconds long. And, according to an embodiment, the time difference between said time interval and said preceding time interval is at least 4 minutes.

These embodiments contribute to the reliability of the proposed solution for determining a person's level of attention. These embodiments ensure that a significant variation is observed in the estimated parameters (i.e., the slope and/or the offset of the 1/f characteristic, and possibly the power of the peak in the alpha band), and allow to reliably detect a fluctuation in the person's attention level. Within the scope of the invention, other implementations of the proposed method could also be envisaged.

According to an embodiment, the neural activity signals comprise at least 3 electroencephalography signals, and preferably 64 electroencephalography signals.

This embodiment proposes using at least 3 electrodes placed on the person's head to obtain electroencephalography signals representative of the person's neural activity. Specifically, the used electrodes comprise: at least 1 electrode for ground (and preferably 2 ground electrodes); at least 1 reference electrode placed on the mastoid (and preferably 2 reference electrodes); and one or more electrodes of interest (over all or part of the cortical regions). Preferably, 64 electrodes placed on the person's head are used to obtain the electroencephalography signals.

According to another aspect, the present invention provides a driving assistance method comprising:

- a step of determining an attention level of a driver of a vehicle using the method conforming to the invention ; and
- a step of performing at least one control action based on the attention level of the driver.

The proposed driving assistance method has the advantages of the previously described method for determining a person's attention level. It allows monitoring the driver's attention level in real-time. In comparison with existing solutions, it can detect rapidly (within 800 milliseconds) fluctuations of the driver's attention, and perform the appropriate control actions in due time. The proposed driving assistance method hence contributes to improving safety and preventing accidents.

According to another aspect, the present invention provides a device for determining at least one attention level of a person based on time signals representative of a neural activity of the person over a plurality of time intervals, the device comprising:

- a module for estimating parameters of power spectra of the neural activity signals over one said time interval, the estimated parameters including power-law exponents and/or offsets of the power spectra; and

- a module for determining a said attention level of the person based on a variation between: the parameters estimated for said time interval; and parameters estimated for a preceding time interval.

The device can be configured to implement each of the embodiments of the method conforming to the invention. In particular, for each step of the method conforming to the invention, the proposed device may comprise a corresponding module configured to implement said step.

According to another aspect, the present invention provides a driving assistance system comprising the device conforming to the invention for determining at least one attention level of a driver of a vehicle. The driving assistance system is configured to perform at least one control action based on the attention level of the driver.

According to another aspect, the invention provides a vehicle comprising: a device conforming to the invention for determining an attention level; or a driving assistance system conforming to the invention.

The present invention can apply to any type of vehicle, including road vehicles (e.g., cars), ships and aircrafts.

According to another aspect, the present invention provides a computer program comprising instructions which, when the program is executed by at least one processor or a computer, cause said at least one processor or computer to implement a method for determining an attention level conforming to the invention, or a driving assistance method conforming to the invention.

The computer program referred to here may use any programming language, and be in the form of source code, object code, or code intermediate between source code and object code, such as in a partially compiled form, or in any other desirable form.

According to another aspect, the present invention provides a computer-readable medium having stored thereon the computer program conforming to the invention.

The computer-readable medium referred to here may be any entity or device capable of storing the program and readable by any computer equipment, including a computer. For example, the medium may include a storage medium, or a magnetic storage medium, such as a hard drive. Alternatively, the storage medium may correspond to a computer integrated circuit in which the program is incorporated and adapted to execute a method as described above or to be used in the execution of such method.

It should be emphasized that the proposed driving assistance method, device for determining an attention level, driving assistance system, vehicle, computer program, and computer-readable medium present the advantages described above in relation with the proposed method for determining an attention level.

BRIEF DESCRIPTION OF THE DRAWINGS

Features, advantages, and technical and industrial significance of exemplary embodiments of the invention will be described below with reference to the accompanying drawings, in which like signs denote like elements, and wherein:

FIG. 1-6 illustrate experimental and simulation results related to the invention;

FIG. 7 illustrates the architecture of a driving assistance system according to an embodiment of the invention;

FIG. 8 illustrates steps of a driving assistance method according to an embodiment of the invention; and

FIG. 9 illustrates steps of a method for determining the attention level of a person according to an embodiment of the invention.

DETAILED DESCRIPTION OF EMBODIMENTS

The following description is structured as follows. First, we describe the inventors' experimental and simulation results on which the invention relies. Second, we present an example application of the invention for implementing a driving assistance system.

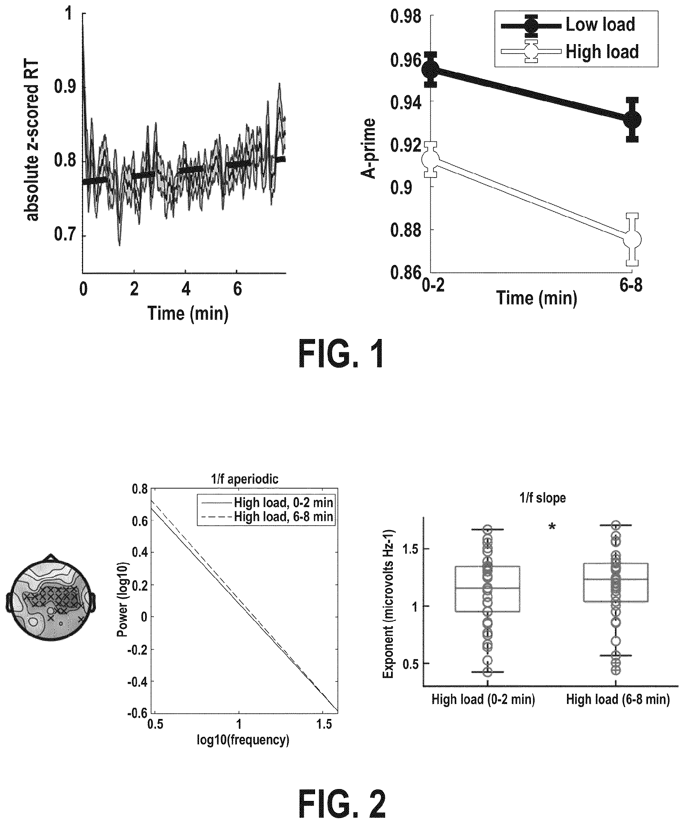

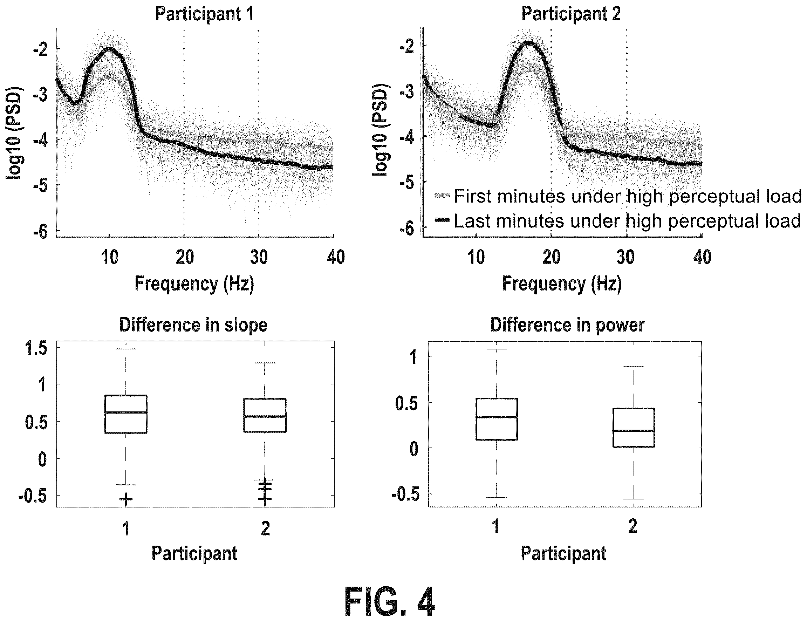

**FIG. 1-6** illustrate experimental and simulation results related to the invention. It should be noted that FIG. 1-3 and 5-6 illustrate experimental results, whereas FIG. 4 illustrates simulation results.

As detailed below, the inventors' experimental results have shown that certain parameters of the power spectra of neural activity signals are particularly relevant markers of sustained attention, and can be used to determine the attention level of a person.

The inventors' experiments evaluated decrements in sustained attention as a function of time on task, considering two levels of perceptual load.

Thirty-four participants were involved in the experiments. The participants had to monitor video streams comprising natural scenes lasting eight minutes. The video streams comprised circular greyscale photographs of mountain and city scenes. These photographs were presented randomly and with a probability of 10% for mountain and 90% for city scenes. The transition between scenes was gradual by applying a linear interpolation of the two images, with a new image every 800 milliseconds. Participants were instructed to discriminate the category of each new image presentation. Participants performed eight streams of high or low perceptual load in total, with four streams for each perceptual load level. Perceptual load was manipulated by adding an overlaid noise mask (19% noise level) in high perceptual streams.

**FIG. 1** illustrates the fluctuation of sustained attention of the participants as a function of the time-on-task.

More specifically, the used behavioral indicators of fluctuations of sustained attention consisted of: momentary fluctuations of the reaction time variability measured with variance time course. The graph on the left of this figure shows that participants have more variable reaction times as the time-on-task increases. This occurs within 2 minutes periods. The significant linear trend (illustrated by the dotted line on the graph) shows the decrement in sustained attention increases with time-on-task. This decrement in sustained attention is also illustrated by the graph on the right of the figure. This graph shows a significant reduction of detection sensitivity (A-prime) in the last two minutes as compared to the first two minutes of the task. In addition, the increase in perceptual load led to a greater decrement in attention, as illustrated by this graph.

The inventors have found neural markers of sustained attention using electroencephalography signals. These signals were collected using set of multiple electrodes placed on the participants' heads. The inventors used time slots (*i.e.*, trials) of 800ms (the duration of a scene presentation) to transform each time series related to the gradual frequent stimulus presentation into the frequency domain. The power spectrum density was estimated for frequencies from 3 to 40 Hz for each electroencephalography signal *(i.e.,* each electrode), each scene slot, and each participant. The results were averaged corresponding to perceptual load (low or high) and time on task (at the start or the end) over time slots (*i.e.*, trials) for each participant. The inventors then performed non-parametric cluster-based permutation tests to determine whether there was a statistical effect of time-on-task on the power spectrum for any cortical region.

The inventors have found that the power spectrum of neural activity signals comprises significant markers for detecting a decrement in attention as a function of time-on-task. We present these markers of attention in reference to FIG. 2-6.

**FIG. 2** illustrates the variation of the power-law exponent of the power spectrum as a function of the time-on-task.

By parameterizing the power spectrum into periodic and aperiodic components, the inventors have shown that: in conditions of high perceptual load, the reduction in accuracy was associated with a variation of the power-law exponent of the 1/f aperiodic component. As clearly illustrated on this figure, the power-law exponent of the 1/f aperiodic component increases with time-on-task. It follows from the above results, that a decrement in sustained attention is associated with a steeper slope of the 1/f aperiodic component. In particular, it has been shown that this difference is most pronounced for the neural activity signals measured in the right central-parietal region.

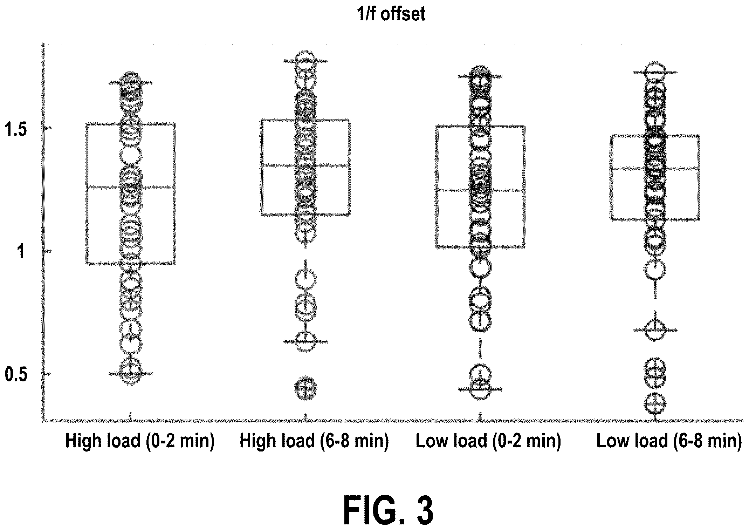

**FIG. 3** illustrates the variation of the offset of the aperiodic component of the power spectrum as a function of the time-on-task.

The inventors have found a main effect of time-on-task with no robust evidence for interaction, such as the offset of the 1/f aperiodic component increases with time-on-task. This result is clearly illustrated on FIG. 3. Therefore, a decrement in sustained attention is associated with a larger offset of the 1/f aperiodic component (regardless of the perceptual load). This difference was related to neural activity signals measured in the right central-parietal region.

**FIG. 4** illustrates simulation results relating to the variation of the power-law exponent of the power spectrum as a function of the time-on-task for different simulated participants.

The simulation results have confirmed that the slope of the 1/f aperiodic component is a particularly reliable marker of attention, since it is unaffected by inter-individual differences related to alpha peak frequency. More specifically, there exists inter-individual variability in the frequency of the peak in the alpha-band (*i.e.*, 8-12 Hz) of the power spectrum. Through a simulation, the inventors have shown that estimating the slope of the 1/f characteristic is more reliable than estimating the raw power of power spectra. This is clearly shown in FIG. 4. Participant 2 has a higher frequency peak for the alpha oscillations, than participant 1 with lower frequency peak for the alpha oscillations. Still, the estimated slope the 1/f aperiodic component is unaffected by these differences, and allows detecting the decrement in attention for both participants.

As can be observed on this figure, the power of the peak in the alpha-band (*i.e.*, 8-12 Hz) of the power spectrum is also a marker of attention decline. This is further illustrated by the following figure.

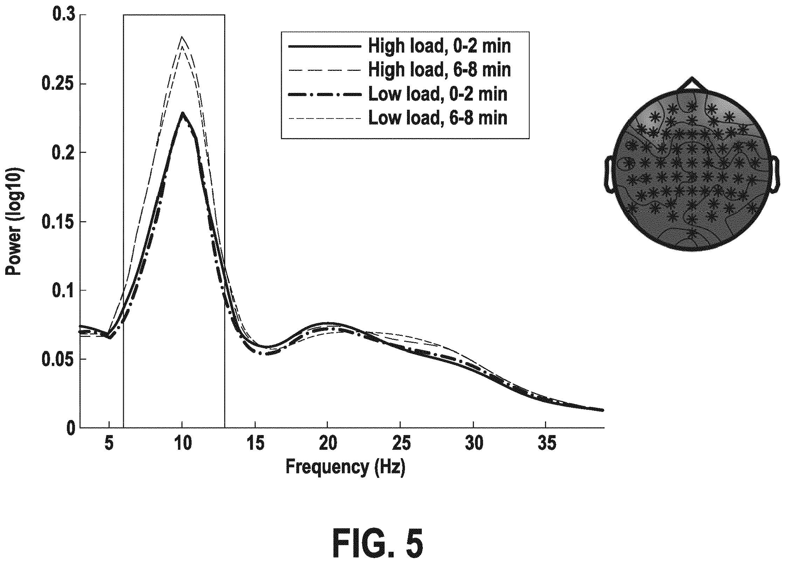

**FIG. 5** illustrates the variation of the power of the peak in the alpha-band of the power spectrum between the first two minutes of the task and the last two minutes of the task.

By parameterizing the power spectrum into aperiodic and periodic components, the inventors have shown that time-on-task had an effect on alpha oscillations, and that this effect was not due to the underlying aperiodic activity. In other words, the aperiodic and periodic components are modelled, and the peak alpha remains. The experimental results show that the power of the alpha oscillations was significantly higher at the end of the task than at its start. This difference was observed across all cortical regions, and was found similar for both levels of perceptual load. These results clearly indicate that a decrement in sustained attention is associated with a larger power of the peak in the alpha-band.

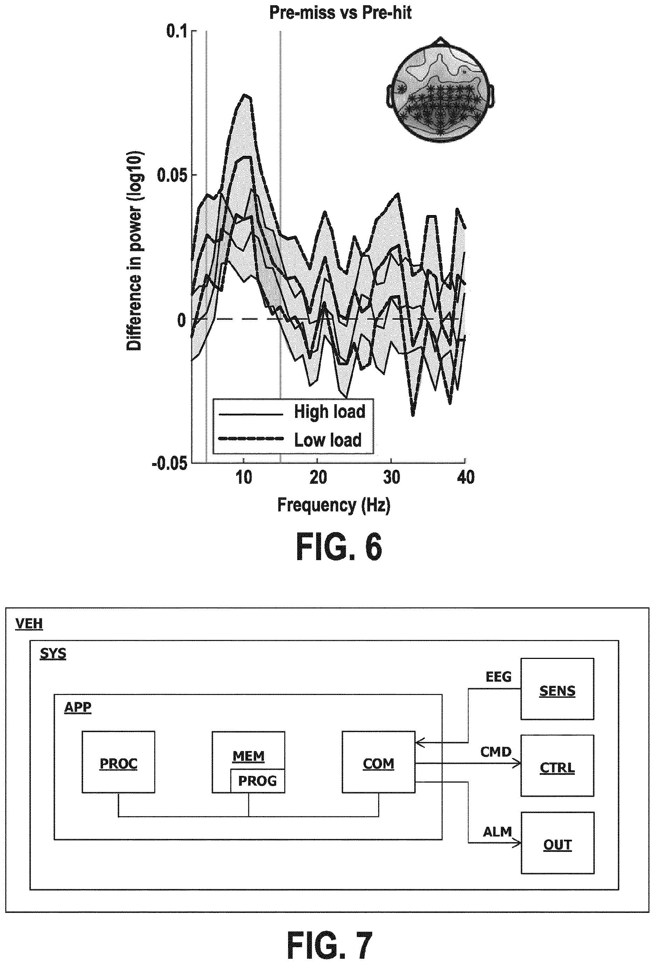

**FIG. 6** illustrates the variation of the power of the peak in the alpha-band of the power spectrum between cases of miss and hit.

The inventors investigated whether the power of the peak in the alpha-band was associated with a greater probability of missing the detection of the target scene (*i.e.*, mountains). To this end, the inventors have analyzed the power spectra for the time slot preceding the target scene in order to compare cases of miss and hit. These analyses have shown that the power of alpha oscillations in the parieto-occipital region was significantly higher in case of miss than in case of hit. This difference was observed for the two levels of perceptual load, and is clearly illustrated by this figure. Thereby, the power of the peak in the alpha-band allows predicting a participant's accuracy in performing the task.

We have described above the experimental and simulation results on which the present invention is based. We now present a particularly advantageous application of the present invention for implementing a driving assistance system.

The following description of the present invention will refer to this particular application, which is provided only as an illustrative example. This example application should not limit the invention which extends to other applications. For instance, the present invention could also be used to determine the attention level of participants in a meeting, or the attention level of operators in a factory.

**FIG. 7** illustrates the architecture of a driving assistance system according to an embodiment of the invention.

Here, a driving assistance system SYS is proposed for monitoring the attention level of a driver of a vehicle VEH. As detailed below, the proposed system SYS uses signals EEG representative of the neural activity of the driver to determine his attention level, and perform control actions.

The driving assistance system SYS of FIG. 7 comprises: a set of sensors SENS; a device APP for determining the attention level of the driver; an output device OUT; and a driving control device CTRL.

The set of sensors SENS is used to obtain the signals EEG representative of the neural activity of the driver. These signals EEG are hereinafter referred to as the *neural activity signals*. In particular, the set of sensors SENS comprises several electrodes placed on the driver's head. For example, 64 electrodes placed on a nylon head cap can be used in combination with two extra bipolar electrodes placed on the left and right mastoids for reference. These electrodes are used to obtain electroencephalography signals EEG (*i.e.,* multiple electroencephalography channels) representative of the neural activity in the different cortical regions of the driver's brain. However, the present invention could also use other types of signals representative of neural activity, such as electrocorticography or magnetoencephalography signals. The set of sensors SENS may further comprise a sensor (e.g., a camera, a lidar, a radar) configured to capture image sequences of the visual driving scene of the vehicle, which can be used to determine the perceptual load level of the driving scene.

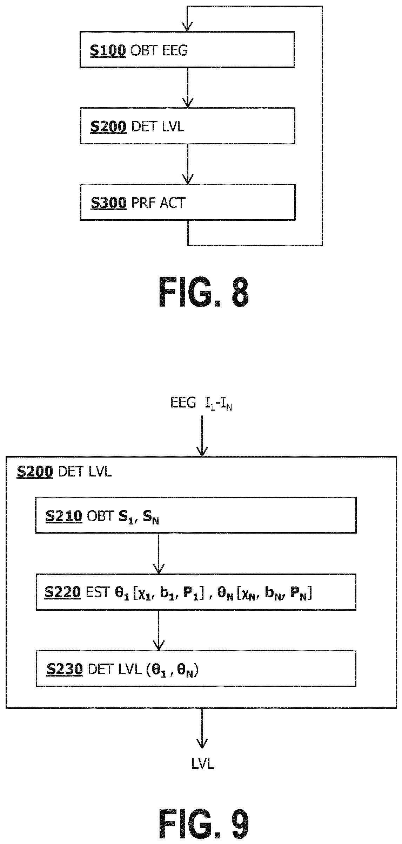

The device APP is configured to determine the driver's attention level using the neural activity signals EEG and, depending on this, perform control actions. For instance, the device APP can send a command CMD to the driving control device CTRL (*e.g.*, an acceleration and/or braking system) based on the driver's attention level. The device APP could also, if the driver's attention level is reduced, alert the driver by sending an alarm signal ALM to the output device OUT (*e.g.*, a display, a speaker, and/or a haptic device). We detail below the hardware architecture of the device APP, and then its operation in reference to FIG. 8.

The device APP of FIG. 7 comprises: at least one processor PROC; and at least one memory MEM.

More specifically, the device APP has, according to an embodiment, the hardware architecture of a computer. The memory MEM constitutes a storage medium conforming to the invention. The memory MEM is readable by processor PROC and stores a computer program PROG conforming to the invention. The computer program PROG comprises instructions for implementing all or part of the steps of a method conforming to the invention, when the computer program PROG is executed by the processor PROC.

As illustrated in this figure, according to an embodiment, the device APP comprises a communication module COM configured to communicate with the set of sensors SENS, the output device OUT, and/or the driving control device CTRL. No limitation is attached to the nature of the communication interfaces between these devices, which can be wired or wireless, and can implement any protocol known to the skilled person.

It should be emphasized that the device APP for determining the attention level of a person based on neural activity signals could be used in systems other than driving assistance systems, such an application should not limit the invention.

The architecture of the driving assistance system SYS having been introduced, we now describe its operation in reference to the following figure.

**FIG. 8** illustrates steps of a driving assistance method according to an embodiment of the invention. Specifically, this figure details an example operation of the driving assistance system SYS previously introduced.

As previously mentioned, the device APP allows monitoring a driver's attention level over time using neural activity signals EEG. To this end, the device APP is configured to implement (all or part of) the steps S100-S300 for each of a plurality of time steps. Each time step is 800 milliseconds long (longer time steps could also be used). We describe below the implementation of these steps considering a current time step.

At step S100, the device APP obtains the time signals EEG representative of the neural activity of the driver over the current time step.

Since the device APP has also implemented step S100 for the preceding time steps, it now has the signals EEG representative of the neural activity of the driver over a plurality of time intervals noted $I_1$-$I_N$. In

particular, each time interval $I_n$ comprises multiple time slots $T_{n,1}$-$T_{n,K}$, each time slot $T_{n,k}$ being associated with one of the time steps and thus being at least 800 milliseconds long.

We remind that the device APP uses a plurality of neural activity signals EEG. In particular, each one of the signals EEG is respectively associated with an electrode measuring the neural activity of the driver.

At step S200, the device APP determines an attention level LVL of the driver based on the neural activity signals EEG over the time intervals $I_1$-$I_N$

The device APP determines the attention level LVL of the driver by comparing the neural activity signals over a time interval $I_N$ with the neural activity signals over a preceding time interval $I_1$. For instance, the device APP compares the neural activity signals over the last two minutes (*i.e.*, the time interval $I_N$) with the neural activity signals over a preceding 2-minute interval (*i.e.*, the time interval $I_1$). It should be noted that, to determine the attention level LVL of the driver, the time difference between the time interval $I_1$ and the time interval $I_N$ must be longer than a specified duration (*e.g.*, 4 minutes).

The implementation of the step S200 is detailed hereinafter in reference to FIG. 9.

At step S300, the device APP performs a control action ACT based on the attention level LVL of the driver determined at the previous step.

For instance, if the attention level LVL is determined to be a reduced attention level, the device APP can alert the driver by sending an alarm signal ALM to the output device OUT. This alarm signal ALM can be an audio signal, a video signal or a haptic signal. Also, the device APP could send a decelerating command CMD to the driving control device CTRL.

Advantageously, it could also be envisaged to adapt a driving interface on a display based on the attention level of the driver. For example, if the driver's attention level is determined to be reduced, the driving interface can highlight detected obstacles (e.g., pedestrians, other cars), lanes, or traffic signs. This allows refocusing the driver's attention on driving, and contributes to improving the user experience.

The proposed solution hence allows the driver's level of attention to be determined at each current time step, up to every 800 milliseconds. Thereby, the proposed solution allows to detect rapidly (within 800 milliseconds) fluctuations in the driver's attention, and to perform in due time the necessary actions to prevent any accident.

We now detail, in reference to the following figure, how the device APP determines the attention level LVL of a person based on the neural activity signals EEG.

**FIG. 9** illustrates steps of a method for determining the attention level of a person according to an embodiment of the invention. This figure details the implementation of the step S200 in which the device APP determines an attention level LVL of a person.

As illustrated in FIG. 9, the step S200 comprises (all or part of) the following steps S210-S230 implemented by the device APP. We detail below the implementation of these steps considering a current time step.

We remind that the device APP determines an attention level LVL of a person by comparing the neural activity signals EEG over the time interval $I_N$ with the neural activity signals EEG over the time interval $I_1$. Here, the time intervals $I_1$ and $I_N$ are respectively at least 2 minutes long, and the time difference between these intervals is at least 4 minutes.

At step S210, the device APP obtains the power spectra $\mathbf{S_N}$ of the neural activity signals EEG over the time interval $I_N$ (*e.g.*, the last two minutes). It also obtains the power spectra $\mathbf{S_1}$ of the neural activity signals EEG over the time interval $I_1$ (e.g., a preceding 2-minute interval). We describe below only the implementation for obtaining the power spectra $\mathbf{S_N}$, but obtaining the power spectra $\mathbf{S_1}$ is similar.

For each one of the neural activity signals EEG, the device APP obtains a power spectrum. We use hereinafter the notation $x_N(f) = (x_N^1(t), ..., x_N^M(t))$ to refer to the different neural activity signals EEG over the time interval $I_N$. The neural activity signals $x_N^1(t), ..., x_N^M(t)$ are respectively transformed into the frequency domain (*e.g.,* by performing a Fast Fourier Transform), and then the power spectrum density is estimated (e.g., for frequencies from 3 to 40 Hz). The device APP thereby obtains the power spectra hereinafter noted $\mathbf{S_N}(f) = (S_N^1(f), ..., S_N^M(f))$.

More specifically, considering a neural activity signal $x_N^m(t)$ over the time interval $I_N$, the power spectrum is estimated for each one of the time slots $T_{N,1}$-$T_{N,K}$ of the time interval $I_N$, and then the power spectrum is averaged over the time interval $I_N$ to obtain the power spectrum $S_N^m(f)$.

At step S220, the device APP estimates parameters $\theta_N$ of the power spectra $\mathbf{S_N}$ for the time interval $I_N$ (*e.g.,* the last two minutes). It also estimates parameters $\theta_1$ of the power spectra $\mathbf{S_1}$ for the time interval $I_1$ (*e.g.,* a preceding 2-minute interval). We describe below the implementation for estimating parameters $\theta_N$, and estimating parameters $\theta_1$ is similar.

The estimated parameters $\theta_N$ comprise power-law exponents $\chi_N$ and offsets $\mathbf{b_N}$ of the power spectra $\mathbf{S_N}$. In addition, these parameters may further include powers $\mathbf{P_N}$ of peaks in the alpha band (*i.e.*, from 8 to 12 Hz) of the power spectra $\mathbf{S_N}$.

For clarification purposes, it should be noted that the estimated parameters $\theta_N$ comprise exactly one power-law exponent $\chi_N^m$ for each power spectrum $S_N^m(f)$ (*i.e.*, the slope of the 1/f characteristic), and likewise for the offset $b_N^m$ and the power of the peak in the alpha band $P_N^m$. In other words, we have here $\theta_N$ comprising $\chi_N = (\chi_N^1, ..., \chi_N^M)$, $\boldsymbol{b}_N = (b_N^1, ..., b_N^M)$, and further $\boldsymbol{P}_N = (P_N^1, ..., P_N^M)$.

Within the scope of the invention, several techniques could be envisaged to estimate the parameters $\theta_N$. We present below the use of the technique referred to as *fitting oscillations & one-over-f* (abbreviated FOOOF). However, other techniques known to the skilled person could also be used to estimate these parameters.

With the FOOOF technique, each power spectrum $S_N^m(f)$ is modelled as a combination of an aperiodic component (*i.e.*, a 1/f characteristic), and zero or more periodic components (*i.e.*, peaks rising above the aperiodic component). In this model, the power-law exponent $\chi_N^m$ is associated to the aperiodic component and characterizes its slope. The offset $b_N^m$ characterizes the offset of the aperiodic component. And, the power $P_N^m$ is associated with a periodic component located in the alpha-band.

The model $\hat{S}_N^m(f)$ of the power spectrum $S_N^m(f)$ is expressed by:

$$\hat{S}_N^m(f) = L_N^m(f) + \sum_{i=1}^{I} G_{N,i}^m(f),$$

where: $\hat{S}_N^m(f)$ is the model of the power spectrum expressed in log-scaling; $f$ is the frequency; $L_N^m(f)$ is the aperiodic component; and $I \geq 0$ is the number of periodic components; and $G_{N,i}^m(f)$ is a periodic component (for example, 1=1).

In this model, the aperiodic component $L_N^m(f)$ is expressed by:

$$L_N^m(f) = b_N^m - \log(k_N^m + f^{\chi_N^m}),$$

with the following parameters: $b_N^m$ the offset of the aperiodic component; $k_N^m$ the knee of the aperiodic component; and $\chi_N^m$ is the power-law exponent of the aperiodic component.

Furthermore, each periodic component $G_{N,i}^m(f)$ is respectively expressed using a Gaussian function as follows:

$$G_{N,i}^m(f) = a_{N,i}^m \cdot \exp\left(-\frac{(f - c_{N,i}^m)^2}{2 \cdot (w_{N,i}^m)^2}\right),$$

The above expression describes each peak in the power spectrum using the following parameters: $a_{N,i}^m$ is the height of the peak above the aperiodic component, $c_{N,i}^m$ is the center frequency of the peak, and $w_{N,i}^m$ is the width of the peak.

The implementation details of this model can be found in Donoghue et al., "Parameterizing neural power spectra into periodic and aperiodic components," Nature Neurosciences 23, 2020. The latter article also outlines the implementation details of the algorithm for parameterizing a power spectrum using this model (*i.e.*, for estimating the parameters of such model based on the measured power spectrum).

We remind that, within the scope of the invention, it could also be contemplated to estimate the parameters of the power spectrum using other models or techniques than the one presented above.

For instance, it could envisaged to estimate the power $P_N^m$ of the peak in the alpha-band of the power spectrum $S_N^m(f)$ by using either: a) the estimated parameter $a_{N,i}^m$ of the periodic component in the alpha-band of the model of the power spectrum $\hat{S}_N^m(f)$; or b) the measured power value in the alpha-band of the power spectrum $S_N^m(f)$.

At step S230, the device APP determines an attention level LVL of the person based on a variation between: the parameters $\theta_N$ estimated for the time interval $I_N$; and parameters $\theta_1$ estimated for the time interval $I_1$.

Variation of the power-law exponent: We remind that the estimated parameters $\theta_N$ comprise power-law exponents $\chi_N$ of the power spectra $\boldsymbol{S_N}$. Thereby, the proposed solution may use a variation of the slope of the 1/f aperiodic component as a marker of attention. As previously detailed in reference to FIG. 2, the inventors have found that: in conditions of high perceptual load (e.g., urban driving scenes), a variation of the slope of the 1/f characteristic with time on task is a particularly relevant marker of a fluctuation of the attention. This marker allows detecting rapidly (within 800 milliseconds) the fluctuations in a person's attention.

More specifically, the device APP may verify at step S230 if the following condition is satisfied:

**C1:** all or part of the power-law exponents $\chi_N$ estimated for the time interval $I_N$ is larger than all or part of power-law exponents $\chi_1$ estimated for the time interval $I_1$, and in particular the driving scene is of high perceptual load (superior to a threshold).

If this condition is verified, the device APP may determine that the attention level LVL of the person is a reduced attention level (*i.e.*, inferior to a preceding attention level, or a reference attention level). Otherwise, the attention level LVL is determined to be a regular (*i.e.*, a standard) attention level.

Various approaches can be used to verify the above condition. For instance, the attention level of the person can be determined to be a reduced attention level if the average of the power-law exponents $\chi_N$ estimated for the time interval $I_N$ is larger than the average of the power-law exponents $\chi_1$ estimated for the time interval $I_1$. However, it could be envisaged to use the sum of the power-law exponents, or even a cluster-based analysis to verify this condition.

As previously mentioned, it is proposed here to determine the person's attention level using jointly a variation between estimated power-law exponents $\chi_N$ and $\chi_1$, and the perceptual load level of the visual scene perceived by the person (*i.e.*, the driving scene). Accordingly, the proposed method may include a step of determining the perceptual load level of the driving scene. For instance, this step can be performed, based on an image sequence representing the driving scene and captured by a sensor (e.g., a camera, a lidar, a radar) aboard the vehicle, using the solution proposed in the international application WO 2020/253965 A1 - this international application is hereby incorporated by reference. Examples of high perceptual load scenes include urban driving scenes, or driving scenes involving vehicles and pedestrians in motion.

In particular, the estimated power-law exponents used to determine the attention level LVL may correspond to the power-law exponents associated with the neural activity signals EEG in the right central-parietal region, rather than the neural activity signals over all cortical regions. This allows determining reliably the attention level of the person using a limited number of neural activity signals. As previously detailed in reference to FIG. 2, the inventors have found that a decrement in sustained attention is associated with a steeper 1/f aperiodic component in conditions of high perceptual load, and that this difference is most pronounced in the right central-parietal region.

Variation of the offset: The estimated parameters $\theta_N$ comprise the offsets $b_N$ of the power spectra $S_N$. It is here proposed to use a variation of the offset of the 1/f aperiodic component as a marker of attention. As previously detailed in reference to FIG. 3, the inventors' experimental results have shown that a variation of the offset of the 1/f aperiodic component with time on task is a particularly relevant marker of a fluctuation of the attention (regardless of the perceptual load). This marker allows detecting rapidly (within 800 milliseconds) the fluctuations in a person's attention.

Specifically, the device APP may verify at step S230 if the following condition is satisfied:

- **C2:** all or part of the offsets $b_N$ estimated for the time interval $I_N$ is larger than all or part of the offsets $b_1$ estimated for the time interval $I_1$.

If this condition is verified, the device APP may determine that the attention level LVL of the person is a reduced attention level.

To verify the above condition, various approaches can be used, such as verifying that the average of the offsets $b_N$ estimated for the time interval $I_N$ is larger than the average of the offsets $b_1$ estimated for the time interval $I_1$. The sum of the offsets, or a cluster-based analysis could also be used.

It is important to note that the offset of the 1/f characteristic can be used as an alternative to, or in combination with, the slope of the 1/f characteristic. In other words, the device APP may determine that the attention level LVL of the person is a reduced attention level if either of conditions **C1** and **C2** is verified. Alternatively, it may determine that the attention level LVL is reduced if both conditions **C1** and **C2** are satisfied, which contributes to improving the reliability of the proposed solution.

Similarly to the estimated power-law exponents, the estimated offsets used to determine the attention level LVL may correspond to the offsets associated with the neural activity signals EEG in the right central-parietal region, rather than the neural activity signals over all cortical regions.

Variation of the power of the peak in the alpha-band: In a particular embodiment, the device APP uses at step S230 jointly the aforementioned attention markers (*i.e.*, the slope and/or the offset of the 1/f characteristic) and the powers of the peaks in the alpha-band $P_N$ to determine the attention level LVL of the person. As previously detailed in reference to FIG. 5, the inventors have shown that a decrement in sustained attention is also associated with a larger power of the peak in the alpha-band (regardless of the perceptual load). This embodiment hence contributes to improving the reliability of the proposed solution for determining the attention level of a person.

Specifically, the device APP may further verify at step S230 that the following condition is satisfied:

**C3**: all or part of the powers of the peaks in the alpha-band $P_N$ estimated for the time interval $I_N$ is larger than all or part of the powers of the peaks in the alpha-band $P_1$ estimated for the time interval $I_1$.

It should be emphasized that the power of the peak in the alpha band is used in combination with the slope and/or the offset of the 1/f aperiodic component. For instance, if conditions **C1** and **C3** are jointly satisfied, the device APP may determine that the attention level of the person LVL is a reduced attention level. Alternatively, if conditions **C2** and **C3** are jointly satisfied, the device APP may determine that the attention level of the person LVL is a reduced attention level.

Advantageously, all of the three markers described above can be used jointly. In other words, the device APP may determine that the attention level of the person LVL is reduced if all of conditions **C1, C2,** and **C3** are jointly satisfied, which contributes to improving the reliability of the proposed solution.

Generally, the powers of peaks in the alpha band estimated across all cortical regions can be used to determine the attention level of the person. Indeed, the inventors have shown that this marker is significantly higher for all cortical regions after a certain period spent on a task by the person as mentioned in reference to FIG. 5.

Nevertheless, the estimated powers of peaks in the alpha band used to determine the attention level LVL may correspond to the powers of peaks in the alpha-band associated with the neural activity signals EEG in the parieto-occipital region. This allows determining reliably the attention level of the person using a limited number of neural activity signals. The inventors have found that the power of alpha oscillations measured by electrodes in the parieto-occipital region significantly predicts a person's ability to perform a task accurately - *cf.* FIG. 6.

**Additional Variants:** Although the present invention has been described above with reference to certain specific embodiments, it will be understood that the invention is not limited by the particularities of these embodiments. Numerous variations, modifications, and developments may be made in the above-described embodiments within the scope of the claims.

**Claims**

1. A computer-implemented method for determining (S200) at least one attention level (LVL) of a person based on time signals (EEG) representative of a neural activity of the person over a plurality of time intervals $(I_1\text{-}I_N)$, the method comprising for at least one said time interval $(I_N)$:

     - a step of estimating (S220) parameters $(\theta_N)$ of power spectra $(S_N)$ of the neural activity signals (EEG) over said time interval $(I_N)$, the estimated parameters $(\theta_N)$ including power-law exponents $(\chi_N)$ and/or offsets $(b_N)$ of the power spectra $(S_N)$; and
     - a step of determining (S230) a said attention level (LVL) of the person based on a variation between: the parameters $(\theta_N)$ estimated for said time interval $(I_N)$; and parameters $(\theta_1)$ estimated for a preceding time interval $(I_1)$.

2. The method of claim 1, wherein said attention level of the person (LVL) is determined (S230) to be a reduced attention level (LVL) if the following condition is satisfied:

     - all or part of the power-law exponents $(\chi_N)$ estimated for said time interval $(I_N)$ are larger than all or part of power-law exponents $(\chi_1)$ estimated for said preceding time interval $(I_1)$, and in particular a visual scene perceived by the person is of high perceptual load.

3. The method of claim 1 or 2, wherein said attention level of the person (LVL) is determined (S230) to be a reduced attention level (LVL) if the following condition is satisfied:

     - all or part of the offsets $(b_N)$ estimated for said time interval $(I_N)$ are larger than all or part of offsets $(b_1)$ estimated for said preceding time interval $(I_1)$.

4. The method of any one of claims 1 to 3, wherein the estimated power-law exponents $(\chi_N)$ and/or offsets $(b_N)$ used to determine (S230) said attention level (LVL) correspond to power-law exponents $(\chi_N)$ and/or offsets $(b_N)$ of power spectra $(S_N)$ of the neural activity signals (EEG) in the right central-parietal region.

5. The method of any one of claims 1 to 4, wherein the estimated parameters $(\theta_N)$ are parameters of models of the power spectra $(S_N)$ such that each power spectrum is modelled as a combination of an aperiodic component and zero or more periodic components, and that the aperiodic component is defined by one of the estimated power-law exponents $(\chi_N)$ and one of the estimated offsets $(b_N)$.

6. The method of any one of claims 1 to 5, wherein the estimated parameters $(\theta_N)$ further include powers of peaks in the alpha band $(P_N)$ of the power spectra $(S_N)$.

7. The method of claim 6, wherein said attention level of the person (LVL) is determined (S230) to be a reduced attention level (LVL) if all of the following conditions are jointly satisfied:

     - all or part of the power-law exponents $(\chi_N)$ estimated for said time interval $(I_N)$ are larger than all or part of power-law exponents $(\chi_1)$ estimated for said preceding time interval $(I_1)$, and in particular a visual scene perceived by the person is of high perceptual load;

- all or part of the offsets ($b_N$) estimated for said time interval ($I_N$) are larger than all or part of offsets ($b_1$) estimated for said preceding time interval ($I_1$); and
- all or part of the powers of the peaks in the alpha-band ($P_N$) estimated for said time interval ($I_N$) are larger than all or part of powers of peaks in the alpha-band ($P_1$) estimated for said preceding time interval ($I_1$).

8. The method of claim 6 or 7, wherein the estimated powers of peaks in the alpha band ($P_N$) used to determine (S230) said attention level (LVL) correspond to powers of peaks in the alpha band ($P_N$) of power spectra ($S_N$) of the neural activity signals (EEG) in the parieto-occipital region.

9. The method of any one of claims 1 to 8, wherein said steps of estimating (S220) and determining (S230) are iterated at each of a plurality of time steps, and wherein each said time step is 800 milliseconds long or more.

10. The method of any one of claims 1 to 9, wherein each said time interval ($I_n$) is at least 2 minutes long and comprises multiple time slots ($T_{n,1}$-$T_{n,K}$), and wherein each said time slot ($T_{n,k}$) is at least 800 milliseconds long.

11. The method of any one of claims 1 to 10, wherein the time difference between said time interval ($I_N$) and said preceding time interval ($I_1$) is at least 4 minutes.

12. The method of any one of claims 1 to 11, wherein the neural activity signals (EEG) comprise at least 3 electroencephalography signals, and preferably 64 electroencephalography signals.

13. A driving assistance method comprising:

- a step of determining (S200) an attention level (LVL) of a driver of a vehicle (VEH) using the method of any one of claims 1 to 12; and
- a step of performing (S300) at least one control action (ACT) based on the attention level (LVL) of the driver.

14. A device (APP) for determining (S200) at least one attention level (LVL) of a person based on time signals (EEG) representative of a neural activity of the person over a plurality of time intervals ($I_1$-$I_N$), the device (APP) comprising:

- a module for estimating (S220) parameters ($\theta_N$) of power spectra ($S_N$) of the neural activity signals (EEG) over one said time interval ($I_N$), the estimated parameters ($\theta_N$) including power-law exponents ($\chi_N$) and/or offsets ($b_N$) of the power spectra ($S_N$); and
- a module for determining (S230) a said attention level (LVL) of the person based on a variation between: the parameters ($\theta_N$) estimated for said time interval ($I_N$); and parameters ($\theta_1$) estimated for a preceding time interval ($I_1$).

15. A driving assistance system (SYS) comprising the device (APP) of claim 14 for determining at least one attention level (LVL) of a driver of a vehicle (VEH), and wherein the driving assistance system (SYS) is configured to perform (S300) at least one control action (ACT) based on the attention level (LVL) of the driver.

16. A vehicle (VEH) comprising: the device (APP) of claim 14; or the driving assistance system (SYS) of claim 15.

17. A computer program (PROG) comprising instructions which, when the program (PROG) is executed by at least one processor (PROC), cause said at least one processor (PROC) to implement the method of any one of claims 1 to 13.

18. A computer-readable medium (MEM) having stored thereon the computer program (PROG) of claim 17.

**Amended claims in accordance with Rule 137(2) EPC.**

1. A computer-implemented method for determining (S200) at least one attention level (LVL) of a person based on time signals (EEG) representative of a neural activity of the person over a plurality of time intervals ($I_1$-$I_N$), the method comprising for at least one said time interval ($I_N$):

- a step of estimating (S220) parameters ($\theta_N$) of power spectra ($S_N$) of the neural activity signals (EEG) over said time interval ($I_N$), the estimated parameters ($\theta_N$) including power-law exponents ($\chi_N$) of the power spectra ($S_N$);

the method being **characterized in that**:

- the method comprises a step of determining (S230) a said attention level (LVL) of the person based on a variation between: the parameters ($\theta_N$) estimated for said time interval ($I_N$); and parameters ($\theta_1$) estimated for a preceding time interval ($I_1$);
- said attention level of the person (LVL) is determined (S230) to be a reduced attention level (LVL) if the following condition is satisfied:

o all or part of the power-law exponents ($\chi_N$) estimated for said time interval ($I_N$) are larger than all or part of power-law exponents ($\chi_1$) estimated for said preceding time interval ($I_1$), and in particular a visual scene perceived by the person is of high perceptual load.

2. The method of claim 1, wherein the estimated parameters ($\theta_N$) further include offsets ($b_N$) of the power spectra ($S_N$), and wherein said attention level of the person (LVL) is determined (S230) to be a reduced attention level (LVL) if the following conditions are jointly satisfied:

    - all or part of the power-law exponents ($\chi_N$) estimated for said time interval ($I_N$) are larger than all or part of power-law exponents ($\chi_1$) estimated for said preceding time interval ($I_1$), and in particular a visual scene perceived by the person is of high perceptual load; and
    - all or part of the offsets ($b_N$) estimated for said time interval ($I_N$) are larger than all or part of offsets ($b_1$) estimated for said preceding time interval ($I_1$).

3. The method of claim 1 or 2, wherein the estimated power-law exponents ($\chi_N$) and/or offsets ($b_N$) used to determine (S230) said attention level (LVL) correspond to power-law exponents ($\chi_N$) and/or offsets ($b_N$) of power spectra ($S_N$) of the neural activity signals (EEG) in the right central-parietal region.

4. The method of any one of claims 1 to 3, wherein the estimated parameters ($\theta_N$) are parameters of models of the power spectra ($S_N$) such that each power spectrum is modelled as a combination of an aperiodic component and zero or more periodic components, and that the aperiodic component is defined by one of the estimated power-law exponents ($\chi_N$) and one of the estimated offsets ($b_N$).

5. The method of any one of claims 1 to 4, wherein the estimated parameters ($\theta_N$) further include powers of peaks in the alpha band ($P_N$) of the power spectra ($S_N$).

6. The method of claims 2 and 5, wherein said attention level of the person (LVL) is determined (S230) to be a reduced attention level (LVL) if all of the following conditions are jointly satisfied:

    - all or part of the power-law exponents ($\chi_N$) estimated for said time interval ($I_N$) are larger than all or part of power-law exponents ($\chi_1$) estimated for said preceding time interval ($I_1$), and in particular a visual scene perceived by the person is of high perceptual load;
    - all or part of the offsets ($b_N$) estimated for said time interval ($I_N$) are larger than all or part of

offsets ($b_1$) estimated for said preceding time interval ($I_1$); and
    - all or part of the powers of the peaks in the alpha-band ($P_N$) estimated for said time interval ($I_N$) are larger than all or part of powers of peaks in the alpha-band ($P_1$) estimated for said preceding time interval ($I_1$).

7. The method of claim 5 or 6, wherein the estimated powers of peaks in the alpha band ($P_N$) used to determine (S230) said attention level (LVL) correspond to powers of peaks in the alpha band ($P_N$) of power spectra ($S_N$) of the neural activity signals (EEG) in the parieto-occipital region.

8. The method of any one of claims 1 to 7, wherein said steps of estimating (S220) and determining (S230) are iterated at each of a plurality of time steps, and wherein each said time step is 800 milliseconds long or more.

9. The method of any one of claims 1 to 8, wherein each said time interval ($I_n$) is at least 2 minutes long and comprises multiple time slots ($T_{n,1}$-$T_{n,K}$), and wherein each said time slot ($T_{n,k}$) is at least 800 milliseconds long.

10. The method of any one of claims 1 to 9, wherein the time difference between said time interval ($I_N$) and said preceding time interval ($I_1$) is at least 4 minutes.

11. The method of any one of claims 1 to 10, wherein the neural activity signals (EEG) comprise at least 3 electroencephalography signals, and preferably 64 electroencephalography signals.

12. A driving assistance method comprising:

    - a step of determining (S200) an attention level (LVL) of a driver of a vehicle (VEH) using the method of any one of claims 1 to 11; and
    - a step of performing (S300) at least one control action (ACT) based on the attention level (LVL) of the driver.

13. A device (APP) for determining (S200) at least one attention level (LVL) of a person based on time signals (EEG) representative of a neural activity of the person over a plurality of time intervals ($I_1$-$I_N$), the device (APP) comprising:

    - a module for estimating (S220) parameters ($\theta_N$) of power spectra ($S_N$) of the neural activity signals (EEG) over one said time interval ($I_N$), the estimated parameters ($\theta_N$) including power-law exponents ($\chi_N$) of the power spectra ($S_N$);

the device being **characterized in that**:

- the device comprises a module for determining (S230) a said attention level (LVL) of the person based on a variation between: the parameters ($\theta_N$) estimated for said time interval ($I_N$); and parameters ($\theta_1$) estimated for a preceding time interval ($I_1$);
- said attention level of the person (LVL) is determined (S230) to be a reduced attention level (LVL) if the following condition is satisfied:

    o all or part of the power-law exponents ($\chi_N$) estimated for said time interval ($I_N$) are larger than all or part of power-law exponents ($\chi_1$) estimated for said preceding time interval ($I_1$), and in particular a visual scene perceived by the person is of high perceptual load.

**14.** A driving assistance system (SYS) comprising the device (APP) of claim 13 for determining at least one attention level (LVL) of a driver of a vehicle (VEH), and wherein the driving assistance system (SYS) is configured to perform (S300) at least one control action (ACT) based on the attention level (LVL) of the driver.

**15.** A vehicle (VEH) comprising: the device (APP) of claim 13; or the driving assistance system (SYS) of claim 14.

**16.** A computer program (PROG) comprising instructions which, when the program (PROG) is executed by at least one processor (PROC), cause said at least one processor (PROC) to implement the method of any one of claims 1 to 12.

**17.** A computer-readable medium (MEM) having stored thereon the computer program (PROG) of claim 16.

FIG. 1

FIG. 2

FIG. 3

**FIG. 4**

FIG. 5

FIG. 6

FIG. 7

**S100** OBT EEG

**S200** DET LVL

**S300** PRF ACT

# FIG. 8

EEG $I_1$-$I_N$

**S200** DET LVL

**S210** OBT $S_1$, $S_N$

**S220** EST $\theta_1$ [$\chi_1$, $b_1$, $P_1$] , $\theta_N$ [$\chi_N$, $b_N$, $P_N$]

**S230** DET LVL ($\theta_1$ , $\theta_N$)

LVL

# FIG. 9

Europäisches
Patentamt

European
Patent Office

Office européen
des brevets

# EUROPEAN SEARCH REPORT

Application Number

EP 23 19 6082

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 116 204 806 A (HUAWEI TECH CO LTD; UNIV TSINGHUA) 2 June 2023 (2023-06-02) * the whole document * | 1-18 | INV. A61B5/18 A61B5/372 |
| A | LIN CHIN-TENG ET AL: "Wireless and Wearable EEG System for Evaluating Driver Vigilance", IEEE TRANSACTIONS ON BIOMEDICAL CIRCUITS AND SYSTEMS, IEEE, US, vol. 8, no. 2, 1 April 2014 (2014-04-01), pages 165-176, XP011549080, ISSN: 1932-4545, DOI: 10.1109/TBCAS.2014.2316224 [retrieved on 2014-05-23] * the whole document * | 1-18 | |
| A | BAILEY NEIL W ET AL: "Mindfulness Meditators Show Enhanced Accuracy and Different Neural Activity During Working Memory", MINDFULNESS, SPRINGER US, BOSTON, vol. 11, no. 7, 18 May 2020 (2020-05-18), pages 1762-1781, XP037182069, ISSN: 1868-8527, DOI: 10.1007/S12671-020-01393-8 [retrieved on 2020-05-18] * the whole document * | 1-18 | |

TECHNICAL FIELDS SEARCHED (IPC)

A61B

-/--

The present search report has been drawn up for all claims

| Place of search | Date of completion of the search | Examiner |
|---|---|---|
| Berlin | 21 February 2024 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**Europäisches Patentamt**
**European Patent Office**
**Office européen des brevets**

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | IMMINK MAARTEN A ET AL: "Resting-state aperiodic neural dynamics predict individual differences in visuomotor performance and learning", HUMAN MOVEMENT SCIENCE, ELSEVIER, AMSTERDAM, NL, vol. 78, 15 June 2021 (2021-06-15), XP086699261, ISSN: 0167-9457, DOI: 10.1016/J.HUMOV.2021.102829 [retrieved on 2021-06-15] * the whole document * | 1-18 | |

TECHNICAL FIELDS SEARCHED (IPC)

The present search report has been drawn up for all claims

| Place of search | Date of completion of the search | Examiner |
|---|---|---|
| Berlin | 21 February 2024 | Pohjamo, Terhi |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                    

& : member of the same patent family, corresponding document

EPO FORM 1503 03.82 (P04C01)

## ANNEX TO THE EUROPEAN SEARCH REPORT
## ON EUROPEAN PATENT APPLICATION NO.

**EP 23 19 6082**

This annex lists the patent family members relating to the patent documents cited in the above-mentioned European search report.
The members are as contained in the European Patent Office EDP file on
The European Patent Office is in no way liable for these particulars which are merely given for the purpose of information.

**21-02-2024**

| Patent document cited in search report | Publication date | Patent family member(s) | Publication date |
|---|---|---|---|
| CN 116204806 A | 02-06-2023 | NONE | |

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2020253965 A1

**Non-patent literature cited in the description**

- **DONOGHUE et al.** Parameterizing neural power spectra into periodic and aperiodic components. *Nature Neurosciences*, 2020, vol. 23